(19) Europäisches Patentamt
European Patent Office
Office européen des brevets

(11) Veröffentlichungsnummer: **0 029 001**
**B1**

(12) # EUROPÄISCHE PATENTSCHRIFT

(45) Veröffentlichungstag der Patentschrift: **21.03.84**

(21) Anmeldenummer: **80810254.5**

(22) Anmeldetag: **18.08.80**

(51) Int. Cl.³: **C 07 D401/12**, A 01 N 43/56, A 01 N 43/50, A 01 N 43/64 // (C07D401/12, 249/08, 233/60, 231/12, 213/64)

(54) **Alpha-Phenoxy-propionyl-Azole, deren Herstellung, sie enthaltende herbizide Mittel und deren Verwendung.**

(30) Priorität: **24.08.79 CH 7727/79**

(43) Veröffentlichungstag der Anmeldung: **20.05.81 Patentblatt 81/20**

(45) Bekanntmachung des Hinweises auf die Patenterteilung: **21.03.84 Patentblatt 84/12**

(84) Benannte Vertragsstaaten: **BE CH DE FR GB IT LI NL**

(56) Entgegenhaltungen:
**EP-A- 0 006 608**
**EP-A- 0 014 880**
**FR-A- 2 278 675**
**FR-A- 2 288 089**

(73) Patentinhaber: **CIBA-GEIGY AG**
**Postfach**
**CH-4002 Basel (CH)**

(72) Erfinder: **Böhner, Beat, Dr.**
**Hügelweg 3**
**CH-4102 Binningen (CH)**
Erfinder: **Rempfler, Hermann, Dr.**
**Brücklismattstrasse 16**
**CH-4107 Ettingen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 029 001 B1

Jouve, 18, rue St-Denis, 75001 Paris, France

Alpha-Phenoxy-propionyl-Azole, deren Herstellung, sie enthaltende herbizide Mittel und deren Verwendung

Die vorliegende Erfindung betrifft neue α-Phenoxypropionyl-Azole mit herbizider Wirkung, Verfahren zu deren Herstellung, Mittel, welche diese Verbindungen als Wirkstoffe enthalten sowie die Verwendung dieser Wirkstoffe und Mittel zur Bekämpfung unerwünschten Pflanzenwuchses.

Die neuen α-Phenoxy-propionyl-Azole entsprechen der Formel I

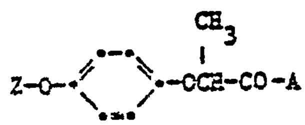

(I)

worin Z den 3,5-Dichlorpyridyl-(2)- oder den 3-Chlor-5-trifluormethyl-pyridyl-(2)-Rest und A einen über ein Ringstickstoffatom gebundenen 1 bis 4 Stickstoffatome enthaltenden Azolrest darstellt, der einfach oder mehrfach durch Halogen, Alkyl-, Alkoxy-, Alkylthio- oder Perfluoralkylreste mit jeweils höchstens 4 C-Atomen, durch Cyan, Carboxy und Carbalkoxyreste mit höchstens 4 C-Atomen in der Alkoxygruppe oder durch $C_1$-$C_4$-Alkanoyl substituiert sein kann, und A auch für Salze von Diazolen und Triazolen stehen kann. Bevorzugter Substituent ist Methyl.

Die genannten Reste Z haben folgende Struktur :

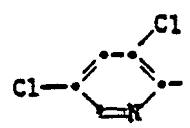

3,5-Dichlorpyridyl-(2)

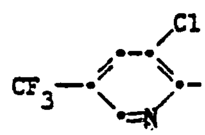

3-Chlor-5-trifluormethyl-pyridyl-(2)

Unter Azolresten werden gegebenenfalls substituierte aromatische 5-Ring-Heterocyclen mit 1 bis 4 Ringstickstoffatomen verstanden, wie Pyrrol, Imidazol, Pyrazol, Triazole (1,2,3-, 1,2,4- und 1,3,4-Triazole), Tetrazol. Diese Azolreste können einfach oder mehrfach durch Halogen, Alkyl-, Alkoxy-, Alkylthio- oder Perfluoralkylreste (wie $CF_3$) mit jeweils bis zu 4 C-Atomen, durch Cyan, Carboxy und Carbalkoxyreste mit bis zu 4 C-Atomen in der Alkoxygruppe oder durch $C_1$-$C_4$ Alkanoyl unabhängig voneinander substituiert sein. Bevorzugte Azolreste A im Molekül sind : Pyrazolyl-1 ; 1,2,4-Triazolyl-1 ; Imidazolyl-1 und 3,5-Dimethylpyrazolyl-1.

Weitere Beispiele substituierter Azole sind : 2,6-Dimethylpyrrol, Tetramethylpyrrol, 3(5)-Methylpyrazol, 4-Methylpyrazol, 3(5)-Aethylpyrazol, 4-Aethylpyrazol, 3(5)-Isopropylpyrazol, 4-Isopropylpyrazol, 2,5-Dimethyl-4-acetylpyrazol, 3,5-Dimethyl-4-propionylpyrazol, 3,4,5-Trimethylpyrazol, 3(5)-Chlorpyrazol, 4-Chlorpyrazol, 4-Brompyrazol, 4-Jodpypazol, 3,4,5-Trichlorpyrazol, 3,4,5-Tribrompyrazol, 3,5-Dimethyl-4-chlorpyrazol, 3,5-Dimethyl-4-brompyrazol, 4-Chlor-3(5)-methylpyrazol, 4-Brom-3(5)-methylpyrazol, 4-Methyl-3,5-dichlorpyrazol, 3(5)-Methyl-4,5(3)-dichlorpyrazol, 3(5)-Chlor-5(3)-methylpyrazol, 4-Methoxypyrazol, 3(5)-Methyl-5(3)-methoxypyrazol, 3(5)-Aethoxy-4,5(3)-dimethylpyrazol, 3(5)-Methyl-5(3)-trifluormethylpyrazol, 3,5-Bistrifluormethylpyrazol, 3(5)-Methyl-5(3)-carbäthoxypyrazol, 3,5-Biscarbäthoxypyrazol, 3,4,5-Triscarbäthoxypyrazol, 3(5)-Methyl-5(3)-methylthio-4-carbäthoxypyrazol, 4-Methyl-3,5-biscarbäthoxypyrazol, 4-Cyanopyrazol, 4-Methoxy-3,5-dichlorpyrazol, 3(5)-Methyl-1,2,4-triazol, 3(5)-Brom-1,2,4-triazol, 3(5)-Chlor-5(3)-methyl-1,2,4-triazol, 3,5-Dichlor-1,2,4-triazol, 2,5-Dibrom-1,2,4-triazol, 3(5)-Chlor-5(3)-cyano-1,2,4-triazol, 3(5)-Chlor-5(3)-carbomethoxy-1,2,4-triazol, 3(5)-Methylthio-1,2,4-triazol, 4(5)-Methyl-1,2,3-triazol, 4,5-Dimethyl-1,2,3-triazol, 4(5)-Chlor-1,2,3-triazol, 1,2,3-Triazol-4(5)-yl-carbonsäureäthylester, 1,2,3-Triazol-4,5-yl-dicarbonsäuredimethylester, 5-Methyltetrazol, 5-Chlortetrazol, Tetrazolyl-5-carbonsäureäthylester.

Darüberhinaus kann der Rest A, wenn das gegebenenfalls substituierte Azol 2 oder 3 Stickstoffatome enthält, auch salzartig an eine der üblichen starken anorganischen oder organischen Säuren, wie Chlorwasserstoffsäure, Bromwasserstoffsäure, Salpetersäure, Schwefelsäure, Tetrafluorborsäure, Fluorsulfonsäure, Ameisensäure, eine halogenierte Carbonsäure, z. B. Trichloressigsäure, eine Alkansulfonsäure, z. B. Methansulfonsäure, eine halogenierte Alkansulfonsäure, z. B. Trifluormethansulfonsäure, Perfluorhexansulfonsäure, eine Arylsulfonsäure, z. B. Dodecylbenzolsulfonsäure, gebunden sein.

Herbizide α-Halogenpyridyl-(2)-oxy-phenoxy-propionylamide, in denen A einen gesättigten N-Heterocyclus oder ein über eine —NH-Brücke gebundenen Triazolrest darstellt, sind schon in der DE-A-2 546 251 (Verbindungen Nos. 57 und 71) bzw. in der US-A-4 046 553 beschrieben worden. Ferner wird in FR-A-2 278 625 ein herbizid wirkendes α-[4-Trifluormethylphenoxy-phenoxy]-propionsäuremorpholid beschrieben.

Die neuen Wirkstoffe der Formel I zeichnen sich gegenüber diesen und anderen vorbekannten Wirkstoffen überraschenderweise durch eine stärkere herbizide Aktivität aus, insbesondere auf monokotyle Unkräuter bei sowohl pre- als auch post-emergenter Applikation. Sie eignen sich deshalb auch hervorragend zur selektiven Bekämpfung monokotyler Unkräuter in gewissen dikotylen Kulturpflanzenbeständen, wie z. B. Soja und Zuckerrübe, aber auch in monokotylen Kulturen wie Weizen. Bevorzugt sind Wirkstoffe der Formel I, in denen Z den 3,5-Dichlorpyridyl-(2)-Rest und A Pyrazol, Imidazol und 1,2,4-Triazol darstellt. Auch Verbindungen mit dem 3-Chlor-5-trifluormethyl-pyridyl-(2)-Rest für Z sind gut wirksam.

Die Verbindungen sind von geringer Toxizität für Menschen und Tiere. Ihre Handhabung kann ohne besondere Vorsichtsmassnahmen erfolgen. Im Feld werden sie vorteilhafterweise in Aufwandmengen von 5 kg/ha und weniger eingesetzt. Sie können im Vorauflaufverfahren eingesetzt werden, Vorrang verdient jedoch häufig der Einsatz nach dem Auflaufen der Kultur.

Die Herstellung der neuen α-Phenoxy-propionyl-azole der Formel I erfolgt mit Vorteil durch Umsetzung eines entsprechenden Propionsäurehalogenids, insbesondere des Chlorids, der Formel II

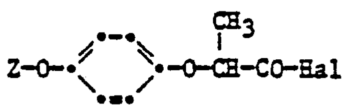

(II)

mit einem 1-Trimethylsilyl-azol der Formel III

$$(CH_3)_3—Si—A \quad (III)$$

worin Z und A wie oben angegeben definiert sind. Dabei entsteht unter Bildung von $(CH)_3Si—Hal$ die Azolverbindung der Formel I.

Diese Umsetzung wird bei Temperaturen zwischen 0° und 100 °C vorzugsweise in einem inerten Lösungsmittel, wie Diäthyläther, Toluol, Dioxan etc. durchgeführt.

Die als Ausgangsstoffe der Formel II dienenden α-Phenoxypropionsäuren und deren Halogenide sind bekannt und z. B. in folgenden Publikationen beschrieben : DE-A-2 531 643, DE-A-2 546 251, GB-A-1 507 643, US-A-4 046 553. Ebenso sind verschiedene Trimethylsilyl-azole der Formel III, wie Trimethylsilyl-pyrazol, Trimethylsilyl-1,2,4-triazol, Trimethylsilyl-imidazol etc. bekannt und beispielsweise in Angew. Chemie 1965, Seite 424 beschrieben worden ; andere lassen sich nach den gleichen Techniken leicht herstellen.

Bei gewissen unsymmetrisch substituierten Azolen, wie Pyrazol, 1,2,3-Triazol, 1,2,4-Triazol etc. treten aufgrund tautomerer Strukturen in den Ausgangsstoffen der Formel III zwei Isomere auf, z. B. beim Pyrazol die Formen

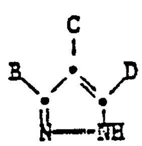

und

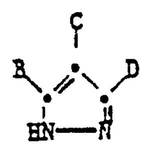

sodass die Verbindungen der Formel I in diesen Fällen auch in zwei isomeren Formen existieren, deren Isomerenverhältnis von der Art der Reste B, C und D bestimmt wird und auf die herbiziden Eigenschaften einen Einfluss haben kann.

Die folgenden Beispiele beschreiben Verfahren zur Herstellung der erfindungsgemässen Verbindungen. Darin sind Temperaturen in Celsiusgraden angegeben.

Weitere hergestellte Wirkstoffe der Formel I sind anschliessend an die Beispiele tabellarisch aufgelistet.

Beispiel 1

17,3 g (0,05 Mol) α-(4-(3′,5′-Dichlorpyridyl-2′-oxy)-phenoxy)-propionsäurechlorid werden bei 10° zu einer Lösung von 7,0 g (0,05 Mol) 1-Trimethylsilyl-pyrazol in 30 ml Toluol zugetropft. Die Temperatur steigt dabei auf 30°. Nach 15 Minuten ist das Ausgangsmaterial aufgebraucht. Das Reaktionsgemisch wird am Rotationsverdampfer bei 55° eingedampft und dann im Hochvakuum getrocknet. Dabei erhält man ein Oel, das beim Anreiben mit Petroläther kristallisiert. Es entstanden 15,9 g (84,1 %) α-(4-(3′,5′-Dichlorpyridyl-2′-oxy)-phenoxy)-propionyl-1-pyrazol mit Fp. 104-106° und der Formel

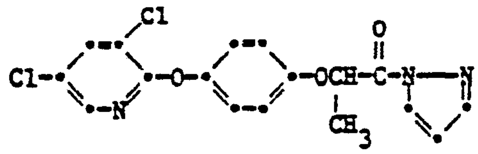

Beispiel 2

17,3 g (0,05 Mol) α-(4-(3',5'-Dichlorpyridyl-2-oxy)-phenoxy)-propionsäurechlorid werden bei 10° zu einer Lösung von 7,1 g (0,05 Mol) 1-Trimethylsilyl-triazol-1,2,4 in 30 ml Toluol zugetropft. Die Temperatur steigt langsam auf 29°. Nach 1/4 Stunde ist kein Ausgangsmaterial mehr vorhanden. Das Reaktionsgemisch wird am Rotationsverdampfer bei 55° eingedampft und im Hochvakuum getrocknet. Dabei erhält man 18,1 g (95,8 %) α-(4-(3',5'-Dichlorpyridyl-2-oxy)-phenoxy)-propionyl-1-triazol-1,2,4 mit Brechungsindex $n_D^{23}$ = 1,583 8 und der Formel

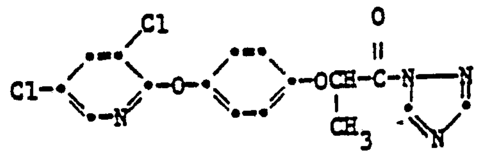

Beispiel 3

17,3 g (0,05 Mol) α-(4-(3',5'-Dichlorpyridyl-2'-oxy)-phenoxy)-propionsäurechlorid werden bei 80° zu einer Lösung von 7,0 g (0,05 Mol) 1-Trimethylsilyl-imidazol in 30 ml Toluol zugetropft. Die Temperatur steigt dabei auf 87 °C. Nach 30 Min. Verbleiben im 100° heissen Bad ist kein Ausgangsmaterial mehr vorhanden. Das Reaktionsgemisch wird am Rotationsverdampfer eingedampft und das zurückbleibende Oel im Hochvakuum getrocknet. Dabei erhält man 16,8 g (88,9 % d. Th.) α-(4-(3',5'-Dichlorpyridyl-2'-oxy)-phenoxy)-propionyl-1-imidazol mit Brechungsindex $n_D^{23}$ = 1,576 3 und der Formel

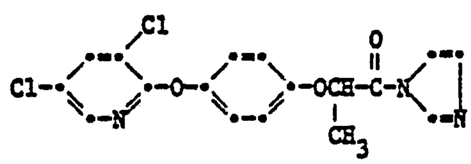

Es sind noch die folgenden Verbindungen der Formel I hergestellt worden.

| Beispiel | Z | A | physikal. Konstanten |
|---|---|---|---|
| 4 | 3-Chlor-5-trifluormethyl-pyridyl-(2) | Pyrazolyl-1 | Fp 70-72°C |
| 5 | " | Imidazolyl-1 | |
| 6 | " | 1,2,4-Triazolyl-1 | Fp 72-74°C |
| 7 | " | 3,5-Dimethyl-pyrazolyl-1 | Fp 118-120°C |

Die neuen α-Phenoxy-propionyl-azole der Formel I sowie die sie enthaltenden Mittel besitzen selbst bei kleinen Aufwandmengen noch eine ausgezeichnete selektiv-herbizide Wirkung gegen monocotyle Unkräuter in verschiedenen Kulturen, vorzugsweise in dikotylen Kulturen.

Ein bevorzugtes Einsatzgebiet ist z. B. die Bekämpfung von grasartigen Unkrautarten in Kulturen wie Baumwolle, Zuckerrüben, Soja und Gemüsekulturen, aber auch in Weizen.

Obwohl die neuen Wirkstoffe der Formel I bei pre- und post-emergenter Anwendung gut wirksam sind, scheint die post-emergente Applikation als Kontaktherbizide den Vorzug zu verdienen, obwohl auch der pre-emergente Einsatz von Interesse ist, besonders bei den Wirkstoffen mit Trifluormethyl-Substituenten.

Die neuen Wirkstoffe werden als z. B. 25 %ige Spritzpulver oder z. B. 20 %ige emulgierbare Konzentrate formuliert und mit Wasser verdünnt, auf die Pflanzenbestände post-emergent appliziert.

Herbizide Wirkung bei Applikation der Wirkstoffe nach dem Auflaufen der Pflanzen (post-emergent)

Verschiedene Kulturpflanzen und Unkräuter werden aus den Samen in Töpfen im Gewächshaus aufgezogen, bis sie das 4- bis 6-Blatt-Stadium erreicht haben. Dann werden die Pflanzen mit wässerigen Wirkstoffemulsionen (erhalten aus einem 20 %igen emulgierbaren Konzentrat) in 3 verschiedenen Dosierungen gespritzt (0,5 ; 0,25 und 0,125 kg Wirkstoff pro Hektar). Die behandelten Pflanzen werden dann bei optimalen Bedingungen von Licht, Begiessung, Temperatur (22-25 °C und Luftfeuchtigkeit 50-70 % relativ) gehalten. 15 Tage nach Behandlung erfolgte die Auswertung des Versuches gemäss folgender Bewertungskala :

1 : Pflanze abgestorben
2-8 : Zwischenstufen der Schädigung
9 : Pflanze gedeiht wie unbehandelte Kontrolle.

Die Verbindungen vorliegender Erfindung sind in diesem Versuch mit den aus der DE-A-No. 2 546 251 Beisp. 57 und 71 bekannten strukturähnlichen α-[4-(3′,5′-Dichlorpyridyl-2′-oxy)-phenoxy]-propionylamiden von N-Heterocyclen verglichen und als wirksamer erkannt worden, wie folgende Resultatzusammenstellung zeigt :

Resultate

Vergleichsverbindung A = DE-A-2 546 251, Beispiel 71

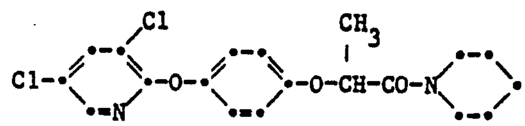

Vergleichsverbindung B = DE-A-2 546 251, Beispiel 57

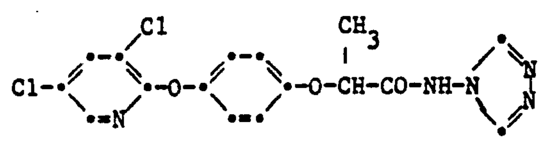

(Siehe Tabelle Seite 6 f.)

| Verbindung Beispiel | Kulturpflanzen Weizen | | | Soja | | | Zuckerrübe | | | Unkräuter Avena fatua | | | Lolium | | | Alopecurus | | | Digitaria | | | Echinochloa | | | Sorghum halopense | | | Rottboellia | | |
|---|---|---|---|---|---|---|---|---|---|---|---|---|---|---|---|---|---|---|---|---|---|---|---|---|---|---|---|---|---|---|
| kg/ha: | 0,5 | 0,25 | 0,125 | 0,5 | 0,25 | 0,125 | 0,5 | 0,25 | 0,125 | 0,5 | 0,25 | 0,125 | 0,5 | 0,25 | 0,125 | 0,5 | 0,25 | 0,125 | 0,5 | 0,25 | 0,125 | 0,5 | 0,25 | 0,125 | 0,5 | 0,25 | 0,125 | 0,5 | 0,25 | 0,125 |
| 1 | 7 | 9 | 9 | 9 | 9 | 9 | 9 | 9 | 9 | 2 | 2 | 4 | 2 | 2 | 3 | 1 | 2 | 2 | 1 | 1 | 2 | 1 | 1 | 1 | 1 | 1 | 2 | 1 | 2 | 2 |
| 2 | 8 | 9 | 9 | 9 | 9 | 9 | 9 | 9 | 9 | 2 | 2 | 4 | 2 | 2 | 3 | 2 | 2 | 2 | 1 | 2 | 2 | 1 | 1 | 1 | 1 | 1 | 3 | 1 | 1 | 1 |
| 3 | 8 | 9 | 9 | 9 | 9 | 9 | 9 | 9 | 9 | 1 | 2 | 2 | 2 | 2 | 2 | 1 | 2 | 3 | 1 | 1 | 1 | 1 | 1 | 1 | 1 | 1 | 1 | 1 | 1 | 2 |
| A | 9 | 9 | 9 | 9 | 9 | 9 | 9 | 9 | 9 | 2 | 3 | 9 | 4 | 8 | 9 | 2 | 3 | 3 | 2 | 3 | 9 | 1 | 1 | 8 | 2 | 4 | 9 | 2 | 6 | 8 |
| B | 9 | 9 | 9 | 9 | 9 | 9 | 9 | 9 | 9 | 2 | 7 | 9 | 3 | 4 | 8 | 2 | 7 | 9 | 2 | 2 | 3 | 1 | 1 | 6 | 3 | 6 | 9 | 1 | 7 | 8 |

Bei praktisch gleich guter Schonung der Kulturpflanzen zeigen die erfindungsgemässen Verbindungen 1 bis 3 eine viel bessere Unkrautwirkung bei niedrigen Aufwandmengen.

Die neuen Wirkstoffe der Formel I sind stabile Verbindungen, welche in üblichen organischen Lösungsmitteln, wie Alkoholen, Aethern, Ketonen, Dimethylformamid, Dimethylsulfoxyd etc. löslich sind.

Die Erfindung betrifft auch herbizide Mittel, welche die neuen Wirkstoffe der Formel I als aktive Komponente enthalten.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden :

Feste Aufarbeitungsformen : Stäubemittel, Streumittel, Granulate, Umhüllungsgsgranulate, Imprägnierungsgranulate und Homogengranulate ;

In Wasser dispergierbare Wirkstoffkonzentrate : Spritzpulver (wettable powder), Paste, Emulsionen ; Emulsionskonzentrate ;

Flüssige Aufarbeitungsformen : Lösungen.

Die Wirkstoffkonzentrationen betragen in den erfindungsgemässen Mitteln 1 bis 80 Gewichtsprozent und können gegebenenfalls bei der Anwendung auch in geringen Konzentrationen wie 0,05 bis 1 % vorliegen.

Die folgenden Formulierungsbeispiele sollen die Herstellung von festen und flüssigen Aufbereitungsformen (Mitteln) näher erläutern.

Emulsionskonzentrat

Zur Herstellung eines 25 %igen Emulsionskonzentrates werden

25 Teile des Wirkstoffes gemäss Beispiel 1,
5 Teile einer Mischung von Nonylphenolpolyoxyäthylen oder Calciumdodecylbenzosulfat,
15 Teile Cyclohexanon,
55 Teile Xylol

miteinander vermischt. Dieses Konzentrat kann mit Wasser zu Emulsionen auf geeigneten Konzentrationen von z. B. 0,1 bis 10 % verdünnt werden. Solche Emulsionen eignen sich zur Bekämpfung von Unkräutern in Kulturpflanzungen.

Granulate

Zur Herstellung eines 5 %igen Granulates werden die folgenden Stoffe verwendet :

5 Teile des Wirkstoffs gemäss Beispiel 4,
0,25 Teile epoxidiertes Pflanzenöl,
0,25 Teile Cetylpolyglykoläther,
3,50 Teile Polyäthylenglykol,
91 Teile Kaolin (Korngrösse : 0,3-0,8 mm).

Die Aktivsubstanz wird mit epoxidiertem Pflanzenöl vermischt und in 6 Teilen Aceton gelöst, hierauf wird Polyäthylenglykol und Cetylpolyglykoläther zugesetzt. Die so erhaltene Lösung wird auf Kaolin aufgesprüht und anschliessend im Vacuum verdampft.

Spritzpulver

Zur Herstellung eines a) 70 %igen und b) 10 %igen Spritzpulvers werden folgende Bestandteile verwendet :

a) 70 Teile des Wirkstoffs gemäss Beispiel 3
5 Teile Natriumdibutylnaphtylsulfonat,
3 Teile Naphthalinsulfonsäuren-Phenolsulfonsäuren-Formaldehyd-Kondensat 3 : 2 : 1,
10 Teile Kaolin,
12 Teile Champagne-Kreide ;

b) 10 Teile des Wirkstoffs gemäss Beispiel 2,
3 Teile Gemisch der Natriumsalze von gesättigten Fettalkoholsulfaten,
5 Teile Naphthalinsulfonsäuren-Formaldehyd-Kondensat,
82 Teile Kaolin.

Die angegebenen Wirkstoffe werden auf die entsprechenden Trägerstoffe (Kaolin und Kreide)

aufgezogen und anschliessend mit den übrigen Bestandteilen vermischt und vermahlen. Man erhält Spritzpulver von vorzüglicher Benetzbarkeit und Schwebefähigkeit. Aus solchen Spritzpulvern können durch Verdünnen mit Wasser Suspensionen von 0,1-80 % Wirkstoff erhalten werden, die sich zur Unkrautbekämpfung in Pflanzenkulturen eignen.

Paste

Zur Herstellung einer 45 %igen Paste werden folgende Stoffe verwendet :

45 Teile des Wirkstoffes gemäss Beispiel 7,
5 Teile Natriumaluminiumsilikat,
14 Teile Cetylpolyglykoläther mit 8 Mol Aethylenoxid,
1 Teil Oleylpolyglykoläther mit 5 Mol Aethylenoxid,
2 Teile Spindeloel,
10 Teile Polyäthylenglykol,
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

**Ansprüche**

1. α-Phenoxy-propionyl-Azole der Formel I

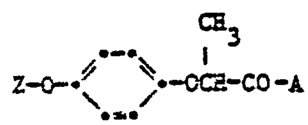

(I)

worin Z den 3,5-Dichlorpyridyl-(2)- oder den 3-Chlor-5-trifluormethyl-pyridyl-(2)-Rest und A einen über ein Ringstickstoffatom gebundenen 1 bis 4 Stickstoffatome enthaltenden Azolrest darstellt, der einfach oder mehrfach durch Halogen, Alkyl-, Alkoxy-, Alkylthio- oder Perfluoralkylreste mit jeweils höchstens 4 C-Atomen, durch Cyan, Carboxy und Carbalkoxyreste mit höchstens 4 C-Atomen in der Alkoxygruppe oder durch $C_1$-$C_4$-Alkanoyl substituiert sein kann, und A auch für Salze von Diazolen und Triazolen stehen kann.

2. α-Phenoxy-propionyl-azole gemäss Anspruch 1, dadurch gekennzeichnet, dass Z den 3,5-Dichlorpyridyl-(2)-Rest darstellt.

3. α-Phenoxy-propionyl-azole gemäss Anspruch 1, dadurch gekennzeichnet, dass Z den 3-Chlor-5-trifluormethyl-pyridyl-(2)-Rest darstellt.

4. α-Phenoxy-propionyl-azole gemäss Anspruch 1, dadurch gekennzeichnet, dass A den Pyrazolyl-1, den Imidazolyl-1, den 1,2,4-Triazolyl-1 oder den 3,5-Dimethyl-pyrazolyl-1-Rest darstellt.

5. Die Verbindung α-[4-(3',5'-Dichlorpyridyl-2'-oxy)-phenoxy]-propionyl-1-pyrazol gemäss den Ansprüchen 2 und 4.

6. Die Verbindung α-[4-(3',5'-Dichlorpyridyl-2'-oxy)-phenoxy]-propionyl-1-triazol-1,2,4 gemäss den Ansprüchen 2 und 4.

7. Die Verbindung α-[4-(3',5'-Dichlorpyridyl-2'-oxy)-phenoxy]-propionyl-1-imidazol.

8. Verfahren zur Herstellung von α-Phenoxy-propionyl-azolen gemäß Anspruch 1, dadurch gekennzeichnet, dass man ein α-Phenoxy-propionsäurehalogenid der Formel II

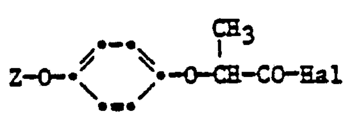

(II)

mit einem 1-Trimethylsilyl-azol der Formel $(CH_3)_3$ Si-A in einem inerten Lösungsmittel umsetzt, wobei « Hal » ein Halogenatom, vorzugsweise Chlor bedeutet und Z und A gemäß Anspruch 1 definiert sind.

9. Verfahren gemäss Anspruch 8, dadurch gekennzeichnet, dass man als Ausgangs-Trimethylsilyl-azole $(CH_3)_3$ Si-A eine der Verbindungen 1-Trimethylsilyl-pyrazol, 1-Trimethylsilyl-imidazol und 1-Trimethylsilyl-1,2,4-triazol verwendet.

10. Herbizides Mittel, dadurch gekennzeichnet, dass es neben inerten flüssigen und/oder festen Verteilungs- und/oder Zuschlagstoffen einschliesslich oberflächenaktiver Stoffe als aktive Komponente ein α-Phenoxy-propionylazol gemäß Anspruch 1 enthält.

11. Herbizides Mittel gemäss Anspruch 10, gekennzeichnet durch den Gehalt an einem Wirkstoff gemäss einem der Ansprüche 5 bis 7.

12. Verwendung der neuen α-Phenoxy-propionylazole gemäß Anspruch 1 oder solche Wirkstoffe

enthaltender Mittel gemäss Anspruch 10 zur Bekämpfung unerwünschten Pflanzenwachstums.

13. Verwendung gemäss Anspruch 12 zur selektiven pre- und vorzugsweise post-emergenten Bekämpfung von insbesondere monocotylen Unkräutern in monocotylen und vorzugsweise dicotylen Kulturpflanzen.

## Claims

1. An α-phenoxypropionylazole of the formula I

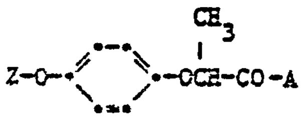 (I)

wherein Z is the 3,5-dichloropyrid-2-yl or 3-chloro-5-trifluoromethyl-pyrid-2-yl radical, and A is an azole radical which contains 1 to 4 nitrogen atoms and is bonded through a ring nitrogen atom and which can be substituted by one or more of halogen, alkyl, alkoxy, alkylthio or perfluoroalkyl, each containing at most 4 carbon atoms, by cyano, carboxyl, and carbalkoxy containing at most 4 carbon atoms in the alkoxy moiety, or by $C_1$-$C_4$ alkanoyl, and A can also denote salts of diazoles and triazoles.

2. An α-phenoxypropionylazole according to claim 1, wherein Z is the 3,5-dichloropyrid-2-yl radical.

3. An α-phenoxypropionylazole according to claim 1, wherein Z is the 3-chloro-5-trifluoromethyl-pyrid-2-yl radical.

4. An α-phenoxypropionylazole according to claim 1, wherein A is the pyrazol-1-yl, imidazol-1-yl, 1,2,4-triazol-1-yl or 3,5-dimethylpyrazol-1-yl radical.

5. α-[4-(3',5'-Dichloropyridyl-2'-oxy)-phenoxy]propion-1-yl-pyrazole according to claims 2 and 4.

6. α-[4-(3',5'-Dichloropyridyl-2'-oxy)-phenoxy]propion-1-yl-1,2,4-triazole according to claims 2 and 4.

7. α-[4-(3',5'-Dichloropyridyl-2'-oxy)-phenoxy]propion-1-yl-imidazole.

8. A process for the production of an α-phenoxypropionylazole according to claim 1, which process comprises reacting an α-phenoxypropionyl halide of the formula II

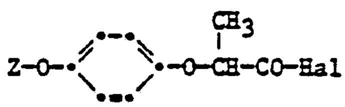 (II)

with a 1-trimethylsilylazole of the formula $(CH_3)_3$-Si-A in an inert solvent, in which formulae Hal is a halogen atom, preferably a chlorine atom, and Z and A are as defined in claim 1.

9. A process according to claim 8, wherein the starting trimethylsilylazole of the formula $(CH_3)_3$-Si-A is 1-trimethylsilylpyrazole, 1-trimethylsilylimidazole or 1-trimethylsilyl-1,2,4-triazole.

10. A herbicidal composition containing a herbicidally effective amount of an α-phenoxypropionylazole according to claim 1, together with one or more inert liquid and/or solid diluents and/or adjuvants, including a surface-active agent.

11. A herbicidal composition according to claim 10 which contains a herbicidally effective amount of a compound as claimed in any one of claims 5 to 7.

12. A method of controlling undesirable plant growth, which comprises applying thereto a herbicidally effective amount of an α-phenoxypropionylazole according to claim 1 or of a composition according to claim 10 wich contains such a compound.

13. A method according to claim 12 for selectively controlling weeds, in particular monocot weeds, pre- and preferably postemergence in crops of monocot and preferably dicot cultivated plants.

## Revendications

1. Alpha-phénoxy-propionyl-azoles de

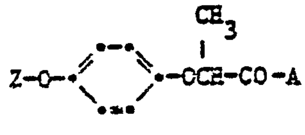 (I)

où Z représente le radical 3,5-dichloropyridyl-(2) ou le radical 3-chloro-5-trifluorométhyl-pyridyl-(2), et A représente un radical azole contenant de 1 à 4 atomes d'azote lié par l'intermédiaire d'un atome d'azote du noyau, qui peut être mono- ou poly-substitué par un halogène, un radical alcoyle, alcoxy, alcoylthio ou

perfluoroalcoyle ayant au plus 4 atomes de carbone chacun, par un cyano, des radicaux carboxy et carbalcoxy ayant au plus 4 atomes de carbone dans le groupe alcoxy ou par un alcanoyle en $C_1$ à $C_4$, et A peut également représenter les sels de diazoles et de triazoles.

2. Alpha-phénoxy-propionyl-azoles selon la revendication 1, caractérisés en ce que Z représente le radical 3,5-dichloropyridyl-(2).

3. Alpha-phénoxy-azoles selon la revendication 1, caractérisés en ce que Z représente le radical 3-chloro-5-trifluorométhyl-pyridyl-(2).

4. Alpha-phénoxy-propionyl-azoles selon la revendication 1, caractérisés en ce que A représente le radical pyrazolyl-1, le radical imidazolyl-1, le radical 1,2,4-triazolyl-1 ou le radical 3,5-diméthyl-pyrazolyl-1.

5. Le composé alpha-(4-(3′,5′-dichloropyridyl-2′-oxy)-phénoxy)-propionyl-1-pyrazole selon les revendications 2 et 4.

6. Le composé alpha-(4-(3′,5′-dichloropyridyl-2′-oxy)-phénoxy)-propionyl-1-triazol-1,2,4 selon les revendications 2 et 4.

7. Le composé alpha-(4-(3′,5′-dichloropyridyl-2′-oxy)-phénoxy)-propionyl-1-imidazole.

8. Procédé de préparation d'alpha-phénoxy-propionyl-azoles selon la revendication 1, caractérisé en ce qu'on fait réagir un halogénure d'acide alpha-phénoxy-propionique de formule II :

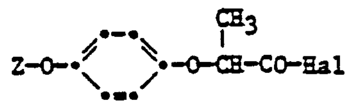

(II)

avec un 1-triméthylsilyl-azole de formule $(CH_3)_3$-Si-A dans un solvant inerte où « Hal » représente un atome d'halogène, de préférence le chlore, et Z et A sont tels que définis selon la revendication 1.

9. Procédé selon la revendication 8, caractérisé en ce qu'on utilise comme triméthylsilyl-azole $(CH_3)_3$-Si-A de départ l'un des composés 1-triméthylsilyl-pyrazole, 1-triméthylsilyl-imidazole et 1-triméthylsilyl-1,2,4-triazole.

10. Agent herbicide caractérisé en ce qu'il contient, outre les dispersants et/ou additifs liquides et/ou solides inertes, y compris les agents tensio-actifs, comme composant actif un alpha-phénoxy-propionyl azole selon la revendication 1.

11. Agent herbicide selon la revendication 10, caractérisé en ce qu'il contient une matière active selon l'une quelconque des revendications 5 à 7.

12. Application des nouveaux alpha-phénoxy-propionyl-azoles selon la revendication 1 ou des agents contenant de telles matières actives selon la revendication 10 pour lutter contre la croissance végétale indésirable.

13. Application selon la revendication 12 à la lutte sélective en pré- et de préférence en post-levée, en particulier contre les mauvaises herbes monocotylédones dans les plantes cultivées monocotylédones et de préférence dicotylédones.